# FASCICULE DE BREVET EUROPEEN

(11) **EP 1 247 144 B1**
(45) Date de publication et mention de la délivrance du brevet: **08.09.2004**
(21) Numéro de dépôt: 01903888.4
(22) Date de dépôt: 08.01.2001
(51) Int. Cl.: G03G 17/00, G01N 21/63, A61B 5/00

(54) **PROCEDE ET INSTALLATION DE DETERMINATION DES PROPRIETES PHYSIQUES D'UN OBJET**
VERFAHREN UND EINRICHTUNG ZUR BESTIMMUNG DER PHYSIKALISCHEN EIGENSCHAFTEN EINES OBJEKTES
METHOD AND INSTALLATION FOR DETERMINING THE PHYSICAL PROPERTIES OF AN OBJECT

(30) Priorité: 10.01.2000 FR 0000241
(43) Date de publication de la demande: 09.10.2002
(73) Titulaire: HERBEPIN, Pascal, 69200 Vénissieux (FR); CANETOS, Jean, 69006 Lyon (FR); Reynes, Jean-Louis, 69150 Décines (FR)
(72) Inventeur: HERBEPIN, Pascal, 69200 Vénissieux (FR); CANETOS, Jean, 69006 Lyon (FR); Reynes, Jean-Louis, 69150 Décines (FR)
(74) Mandataire: Myon, Gérard
(86) Numéro de dépôt international: PCT/FR2001/000047
(87) Numéro de publication internationale: WO 2001/051998

(56) Documents cités:
- DE-A- 3 605 104
- FR-A- 2 410 467
- FR-A- 2 781 046
- US-A- 3 994 283

## Description

L'invention a trait à un procédé et à une installation de détermination des propriétés physiques d'un objet.

Les propriétés physiques d'un objet peuvent être déterminées en étudiant des phénomènes d'ionisation à la surface ou au voisinage de cet objet lorsqu'il est soumis à un potentiel électrique différent d'un élément conducteur placé à proximité.

Par le document FR-A-2 410 467, il est connu d'utiliser la photographie par effet Kirlian dans un but de diagnostic sur le corps humain. Les variations dans la composition du papier photographique, dans son humidité ou dans la composition des bains révélateurs sont telles que les résultats obtenus par la photographie Kirlian sur un support photosensible ne sont généralement pas reproductibles. En outre, ce procédé connu implique de travailler en chambre noire et sa mise en oeuvre est relativement longue dans la mesure où les résultats ne sont visibles qu'après développement des supports photo-sensibles.

Les documents US-A-3, 994,283 et DE-A-44 47 325 divulguent des dispositifs permettant d'observer des phénomènes d'ionisations périphériques par effet Kirlian à la périphérie d'organismes vivants ou d'objets. Ces dispositifs ne permettent pas de caractériser précisément les objets étudiés dans la mesure où les décharges observées présentent une répartition spatiale aléatoire et ne sont pas reproductibles avec une précision satisfaisante.

C'est à ces inconvénients qu'entend plus particulièrement remédier l'invention en proposant un procédé et une installation qui permettent la détermination, en temps réel ou en différé, des propriétés physiques d'un objet avec une excellente reproductibilité, de sorte que des études comparatives systématiques peuvent être envisagées.

Dans cet esprit, l'invention concerne un procédé de détermination des propriétés physiques d'un objet au cours duquel :
- on dispose cet objet au contact d'un capteur dont un élément conducteur est électriquement connecté à un générateur de tension ;
- on applique une tension alternative entre cet objet et cet élément ;
- on visualise et/ou on enregistre un signal optique représentatif de la quantité de lumière d'ionisation d'au moins un gaz au voisinage de cet objet ;
caractérisé en ce qu'il consiste à :
- augmenter progressivement la tension précitée jusqu'à une valeur finale où une luminance maximum est observée ,
- déterminer, comme première valeur caractéristique de cet objet, la valeur de la tension à partir de laquelle la luminance du signal est supérieure ou égale à environ 10% de la luminance maximum et
- déterminer, comme seconde valeur caractéristique de cet objet, la valeur de la tension à partir de laquelle la luminance du signal est supérieure ou égale à environ 90% de la luminance maximum.

L'application de la tension entre l'objet et le corps conducteur peut avoir lieu en mode différentiel ou en mode commun.

Grâce à l'invention, la détermination des premières et secondes valeurs caractéristiques de tension permet de caractériser chaque objet car ces valeurs de tension sont représentatives du comportement de chaque objet et leur détermination ne dépend pas de la forme et/ou de la répartition des phénomènes d'ionisation observés.

Selon un premier aspect avantageux de l'invention, on augmente la tension par paliers, chaque palier correspondant à l'acquisition d'une image du signal, par exemple au moyen d'une ou deux caméras synchronisées.

Selon un autre aspect avantageux, le procédé consiste également à déterminer, comme autre valeur caractéristique de cet objet, un angle de déphasage entre la variation de luminance et la variation de tension dans le temps, cet angle étant défini comme la différence de pente entre une première droite représentative de l'augmentation de tension dans le temps et une seconde droite représentative d'une évolution linéaire de la luminance dans le temps entre des instants où la luminance a des valeurs respectivement égales à environ 10% et environ 90% de la luminance maximum.

Selon un autre aspect avantageux de l'invention, le procédé consiste à augmenter la tension par paliers et à faire varier la fréquence de cette tension sur chaque palier. Ceci permet de rechercher la résonnance énergétique de l'objet étudié. En effet, un objet peut être modélisé sur le plan électrique comme un réseau de circuits de type RLC se présentant en une organisation pseudo-anarchique. Chaque réseau unitaire de type RLC possède une fréquence de résonnance propre et un tel réseau réagit, c'est-à-dire oscille, à certaines fréquences, une telle réaction déterminant les propriétés électriques intrinsèques de l'objet. En d'autres termes, la variation de fréquence permet, pour chaque valeur de tension, de rechercher la fréquence de résonnance à la tension considérée, la résonnance énergétique étant repérée par l'apparition ou l'augmentation des phénomènes d'ionisation obtenus. On peut en outre prévoir de déterminer, en tant qu'autre valeur caractéristique de l'objet, la valeur de fréquence qui correspond à une luminance relative maximum lorsque la fréquence varie, alors que la tension est maintenue à une valeur de palier correspondant à une luminance intermédiaire, notamment égale à environ 50% de la luminance maximum.

Le procédé consiste avantageusement à diviser une surface de mesure définie autour de l'objet sur le capteur en des zones individuelles de mesure et à déterminer les valeurs caractéristiques précitées pour chaque zone individuelle de mesure. Cet aspect de l'invention permet de caractériser différemment les différents bords de l'objet correspondant chacun à une zone ou un secteur de mesure individualisé.

Dans le cas d'une application à la détermination des propriétés d'un corps humain ou animal, on peut appliquer simultanément les dix doigts d'un sujet sur au moins un capteur et ses dix orteils sur un autre capteur, alimenter les éléments conducteurs de ces capteurs en mode différentiel à partir du générateur et déterminer les valeurs caractéristiques précitées concomitamment pour les dix doigts et les dix orteils. Le caractère concomitant de la détermination des valeurs caractéristiques pour les vingt extrémités évite que l'application d'une différence de potentiel pour la détermination de premières valeurs, par exemple relatives à un doigt, ne modifie la structure électrique du sujet avant la détermination d'autres valeurs caractéristiques.

L'invention concerne également une installation destinée à la mise en oeuvre d'un procédé tel que précédemment décrit et, plus spécifiquement, une installation comportant un capteur comprenant une plaque électriquement isolante et un élément électriquement conducteur, des moyens d'alimentation électrique de l'élément électriquement conducteur et de l'objet avec une tension alternative variable et des moyens de visualisation et/ou d'enregistrement à travers la plaque et l'élément conducteur, d'un signal optique représentatif de la quantité de lumière d'ionisation dudit objet , du fait de la tension appliquée. Cette installation est caractérisée en ce que le capteur comprend une membrane souple délimitant, avec la plaque précitée, un volume de confinement d'un gaz ou d'un mélange gazeux apte à s'ioniser sous l'effet de la tension précitée, en ce que ledit capteur est relié à un générateur de façon à creer une tension entre ledit objet et ledit élément conducteur et en ce qu'il est prévu des moyens de traitement dudit signal optique et de commande dudit générateur arrangés pour la mise en oeuvre d'un procédé revendiqué.

Grâce à l'invention, le capteur permet de contrôler la nature exacte du gaz qui s'ionise puisque la composition du gaz ou du mélange gazeux confiné peut être prédéterminée, la tension d'apparition habituelle des phénomènes d'ionisation pouvant être pré-établie, en fonction des caractéristiques physiques des gaz employés.

Selon des aspects avantageux mais non obligatoires de l'invention, l'installation incorpore une ou plusieurs des caractéristiques suivantes :
- la membrane souple est opaque, ce qui permet d'utiliser le capteur à la lumière du jour, l'ionisation pouvant être visualisée et/ou enregistrée dans une zone isolée de la lumière ambiante par la membrane opaque.
- le gaz ou mélange gazeux est emprisonné dans une mousse de matière plastique déformable.
- l'installation comprend des moyens de reconnaissance de forme, des moyens de quantification de la luminance du signal observé, des moyens de commande des moyens d'alimentation électrique, des moyens de détermination automatique de valeurs caractéristiques de l'objet, des moyens de comparaison avec des valeurs de référence de ces valeurs caractéristiques et/ou des moyens de visualisation des résultats de cette détermination ou de cette comparaison.

L'invention sera mieux comprise et d'autres avantages de celle-ci apparaîtront plus clairement à la lumière de la description qui va suivre de deux modes de réalisation d'une installation conforme à son principe et de son procédé de mise en oeuvre, donnée uniquement à titre d'exemple et faite en référence aux dessins annexés dans lesquels :
- la figure 1 est une représentation schématique de principe d'une installation conforme à l'invention en cours d'utilisation ;
- la figure 2 est une coupe partielle de principe d'un capteur utilisé dans l'installation de la figure 1 ;
- la figure 3 est une coupe partielle selon la ligne III-III à la figure 2 ;
- la figure 4 est une représentation des variations dans le temps de la tension appliquée dans l'installation des figures 1 à 3 et de la luminance ;
- la figure 5 est une représentation des variations de luminance lors d'une étape du procédé de l'invention ;
- la figure 6 est un synoptique logique de la partie de traitement des signaux optiques utilisée dans l'installation des figures 1 à 3 ;
- la figure 7 est une vue analogue à la figure 2 pour une installation conforme à un second mode de réalisation de l'invention.

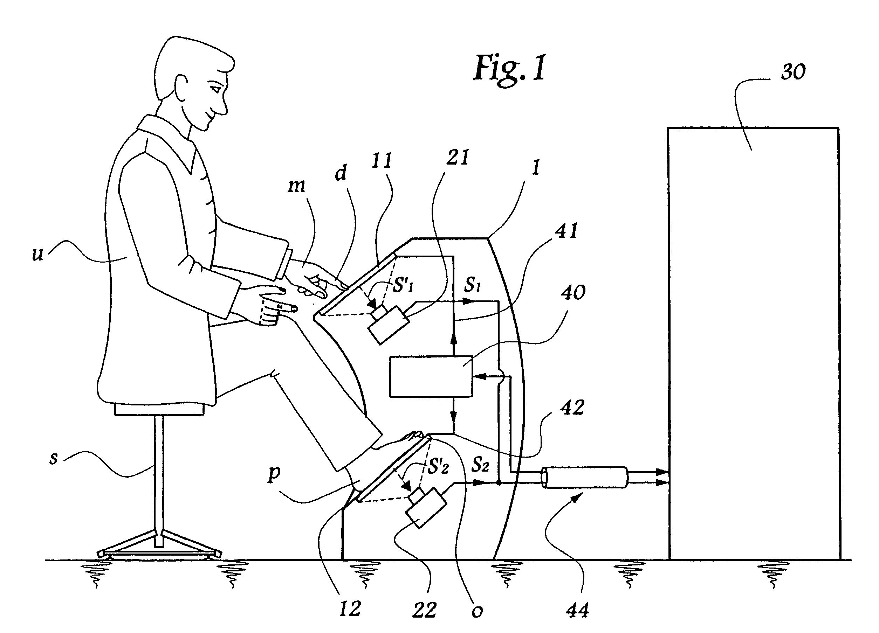

L'installation, représentée aux figures 1, 2, 3 et 6 comprend un boîtier 1 équipé de deux capteurs 11 et 12 disposés au niveau de l'enveloppe du boîtier 1, de telle sorte qu'un utilisateur U assis sur un siège S peut poser ses mains sur le capteur 11 et ses pieds sur le capteur 12.

Deux caméras 21 et 22 équipées de matrices de type CCD sont respectivement disposées à l'arrière des capteurs 11 et 12 par rapport aux extrémités des membres de l'utilisateur. Chaque matrice CCD forme un réseau de cellules photo-sensibles qui génèrent un signal dont la tension est proportionnelle à la luminosité de la partie du signal S'₁ ou S'₂ qu'elles perçoivent à partir du capteur 11 ou 12. Par exemple, dans le cas d'une résolution à 8 bits, la tension de sortie de chaque cellule varie linéairement de 0 à 255 du noir au blanc, ce qui est couramment dénommé "échelle de luminance" dans les domaines de l'informatique ou de la télévision. La luminance est parfois symbolisée dans les systèmes vidéo ou informatiques par la variable Y. Les caméras 21 et 22 sont aptes à fournir à une unité de traitement 30 des signaux vidéo S₁ et S₂ représentatifs de signaux optiques S'₁ et S'₂ qu'elles perçoivent, en particulier de la luminance de ces signaux.

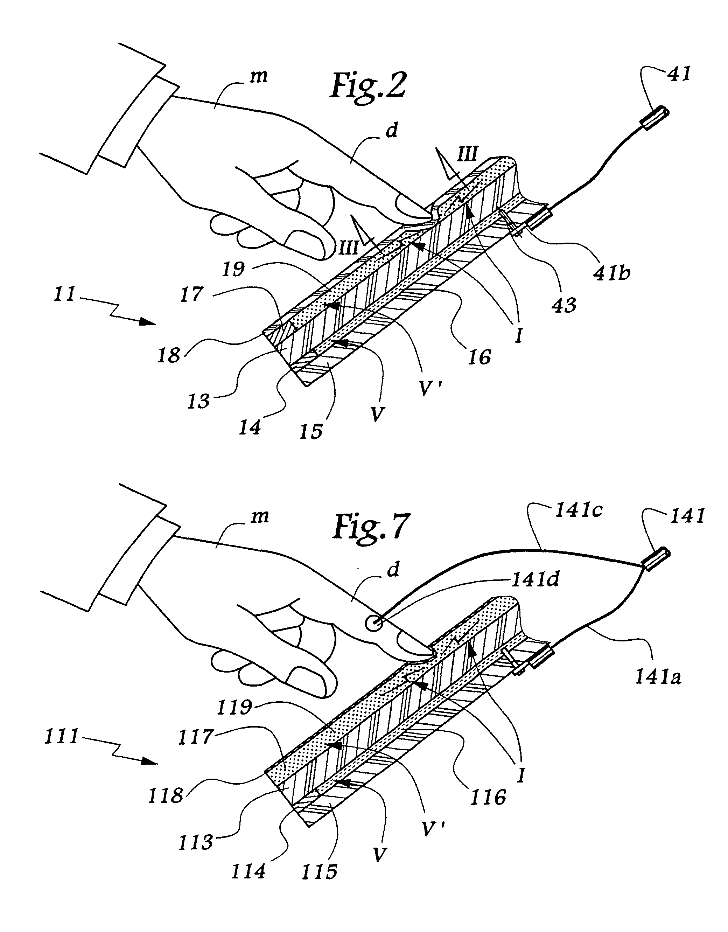

Les capteurs 11 et 12 sont identiques et le capteur 11, qui apparaît partiellement à la figure 2, comprend une plaque isolante 13 réalisée par exemple en matière plastique. Une entretoise 14 permet de définir entre la plaque 13 et une seconde plaque isolante 15, un volume V de réception d'un électrolyte liquide 16, transparent ou translucide, tel qu'un gel du type utilisé en électro-cardiographie ou pour des échographies ou un savon liquide. Les plaques 13 et 15 peuvent être réalisées en verre ou en matière plastique du type polymétacrylate de méthyle ou polyacrylique.

Une seconde entretoise 17 est disposée sur le bord de la plaque 13 à l'opposé du volume V et soutient une membrane souple opaque 18 réalisée dans une feuille d'élastomère, tel que du caoutchouc, synthétique ou naturel, ou du silicone. Entre la plaque 13 et la membrane 18 est défini un volume V' de réception d'un mélange gazeux 19 incluant de l'argon, du néon et du gaz carbonique. Ces gaz présentent la propriété remarquable de donner lieu à des phénomènes d'ionisation I de couleurs différentes, à savoir bleu pour l'argon, orange pour le néon et verte pour le gaz carbonique, ces phénomènes d'ionisation ayant lieu à des potentiels d'ionisation différents et prédéterminés. Ces phénomènes d'ionisation sont obtenus par déplacement de charges électriques sous l'effet de la différence de potentiel entre l'objet, c'est-à-dire par exemple le ou les doigts, et l'électrolyte 16, ces déplacements de charges donnant naissance à des photons. Ces phénomènes d'ionisation se produisent à des valeurs de tension inférieures au valeurs de seuil d'amorçage ou de décharge des gaz considérés, de telle sorte que le système de l'invention n'induit pas la création d'arcs ou de décharges électriques potentiellement dangereux.

Un générateur de tension alternative variable 40 est relié aux électrolytes 16 des capteurs 11 et 12 par des liaisons électriques 41, respectivement 42. Les liaisons 41 et 42 permettent d'appliquer entre ces électrolytes 16 et à travers le corps de l'utilisateur une tension alternative variable U dont on note f la fréquence. Ainsi, compte tenu de l'impédance du corps de l'utilisateur, on crée, par ce mode différentiel, une différence de tension, d'une part, entre les doigts d de l'utilisateur et l'électrolyte 16 du capteur 11 et, d'autre part, entre ses pieds p ou ses orteils o et l'électrolyte 16 du capteur 12. Le conducteur formant la ligne 41 est connecté à une cosse 41b en appui contre la surface de la plaque 15 opposée au volume V et maintenue en position par une vis métallique 43 qui traverse de part en part la plaque 15, de sorte qu'elle est en contact électrique avec l'électrolyte 16.

En pratique, l'utilisateur peut poser ses deux mains m sur le capteur 11 et ses deux pieds p sur le capteur 12, vingt zones d'ionisation potentielle étant contrôlées, qui correspondent respectivement aux positions des extrémités des doigts et des orteils de l'utilisateur.

Comme la membrane 18 est souple, la pression exercée par le doigt d de l'utilisateur permet de déformer cette membrane au point de chasser le mélange gazeux se trouvant entre la membrane 18 et la plaque 13 au niveau du doigt. Le mélange gazeux est ainsi réparti autour du doigt et est susceptible de s'ioniser lorsque la différence de tension U est comprise dans une plage prédéterminée qui dépend de la nature du mélange 19. Ainsi, le volume de gaz ou de mélange gazeux 19 utilisé dans le capteur 11 n'est pas très important, ce qui est significatif sur le plan économique et en ce qui concerne la sécurité de l'utilisateur et de l'opérateur.

Le caractère opaque de la membrane 18 permet aux caméras 21 et 22 de détecter efficacement les phénomènes d'ionisation I dès leur apparition car ceux-ci se produisent dans le spectre visible, alors que les caméras sont isolées de la lumière extérieure par le boîtier 1 et la membrane 18.

Le fonctionnement est le suivant :

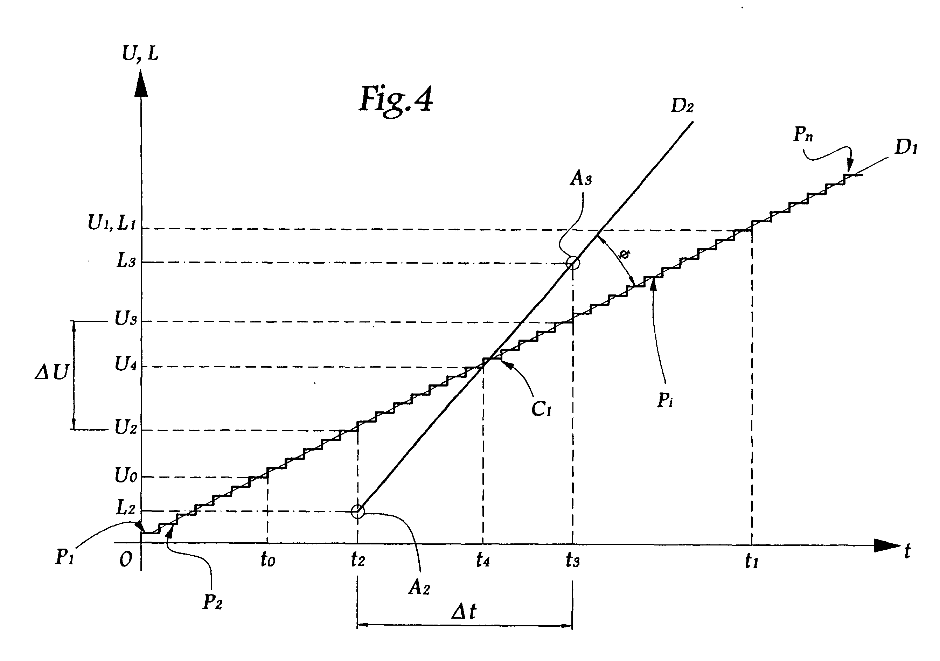

A partir d'un instant initial 0 on augmente progressivement la tension U par paliers P₁, P₂... Pᵢ ... P_{N}, comme représenté par la courbe C₁ à la figure 4. Les points centraux des différents paliers de cette courbe peuvent être reliés par une droite D₁ qui peut être considérée comme une approximation de la courbe C₁.

La montée en tension le long de la courbe C₁ est synchronisée avec la vitesse d'acquisition des caméras 21 et 22. Par exemple, dans le cas de caméras vidéo fonctionnant à 25 images/seconde, la durée de chaque palier Pᵢ est de 40ms, un palier Pᵢ étant ainsi visualisé grâce à deux trames d'image sur chaque signal S₁ ou S₂.

A partir d'un instant auquel la tension U a une valeur U₀, la caméra 21 ou 22 associée au capteur en question détecte l'apparition de lumière issue de l'ionisation et la luminance L du signal optique S'₁ ou S'₂ devient non nulle. La luminance est définie comme l'intensité lumineuse du signal S'₁ ou S'₂ observé.

Lorsque la tension U continue à augmenter, la luminance du signal S'₁ ou S'₂ augmente car les phénomènes d'ionisation I sont de plus en plus nombreux.

La tension U augmente ainsi jusqu'à une valeur U₁ à partir de laquelle une luminance maximum L₁ est obtenue. On note t₁ l'instant où la tension U atteint la valeur U₁.

On détermine alors deux valeurs L₂ et L₃ égale respectivement à 10% et à 90% de la valeur L₁. Sur la base des données enregistrées par l'unité 30 à partir des signaux S₁ est S₂, on détermine les tensions U₂ et U₃ pour lesquelles la luminance est respectivement égale à L₂ et L₃. Ces valeurs U₂ et U₃ sont des valeurs caractéristiques du doigt d considéré car il s'avère en pratique qu'elles peuvent être déterminées de façon reproductible pour un même doigt et dans des conditions opératoires identiques.

Sur la base des enregistrements des signaux S₁ ou S₂, il est possible de déterminer les instants t₀, t₁, t₂ et t₃ auxquels ont été respectivement atteintes les valeurs U₀ à U₃ de la tension U. On note Δt l'écart entre les instants t₂ et t₃ . On note ΔU l'écart entre les valeurs de U₂ et U₃. La pente de la droite D₁ est égale à ΔU/Δt.

Il est possible d'établir, sur une figure du type de la figure 4, et après avoir fixé les échelles de tension et de luminance en ordonnées, une courbe représentative de l'évolution de la luminance en joignant les points A₂ et A₃ définis comme les points où la luminance est respectivement égale à L₂ ou L₃ aux instants t₂ ou t₃. Ces points représentent l'évolution de la luminance de la valeur L₂ à la valeur L₃ sur un intervalle de temps Δt. On note D₂ une droite passant par les points A₂ et A₃ dont la pente est égale à (L₃-L₂)/Δt.

On note Φ l'angle de décalage de pente entre les droites D₁ et D₂. Cet angle peut être considéré comme un indice énergétique caractéristique de l'objet étudié, en l'occurrence le doigt d de l'utilisateur.

Lors de chaque palier de tension Pᵢ de la courbe C₁, la fréquence f de la tension U est modulée par le générateur 40 dans une plage de fréquences comprise entre environ 1000 Hz et environ 800 kHz.

Ainsi, pour chaque tension U appliquée, on teste successivement les différentes fréquences susceptibles de faire résonner le circuit électrique équivalent de l'objet étudié. Ce test est rapide car il a lieu pendant la durée de chaque palier Pᵢ qui peut être de l'ordre de 40 ms comme indiqué ci-dessus.

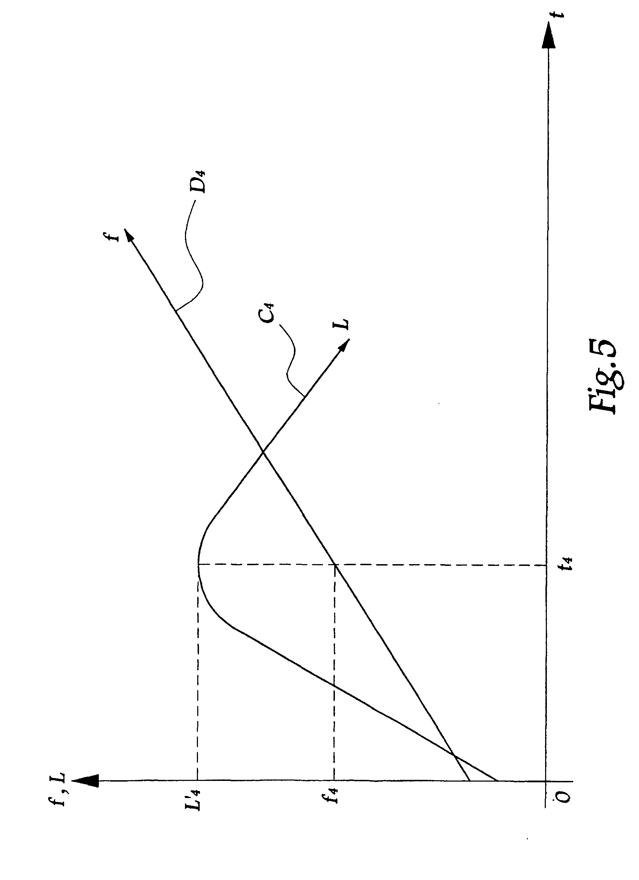

Comme il ressort de la figure 5, si la fréquence f évolue de façon régulière au cours du temps comme représenté par la droite D₄, la luminance observée évolue de façon variable comme représenté par la courbe C₄.

On définit une valeur L₄ de luminance égale à environ 50% de la valeur L₁ et l'on note U₄ la valeur de tension correspondante obtenue au même instant t₄ que la luminance L₄. Sur le palier de tension correspondante, on fait varier continûment la fréquence f de la tension U₄ délivrée par le générateur 40 et l'on observe la variation de la luminance telle que représentée par la courbe C₄. La luminance croît progressivement jusqu'à atteindre, à un instant t₄, une valeur maximum L'₄ relative pour la tension U₄ ; ensuite la luminance décroît. On repère alors la fréquence f₄ correspondant à la valeur maximum L'₄ et l'on définit cette fréquence f₄ comme la fréquence énergétique de l'objet étudié, c'est-à-dire la fréquence à laquelle une luminance relative maximum L'₄ est obtenue, ce qui peut être assimilé à une fréquence de résonnance électromagnétique.

I1 est également possible de déterminer la fréquence énergétique pour plusieurs niveaux de tension intermédiaire entre les valeurs U₀ et U₁.

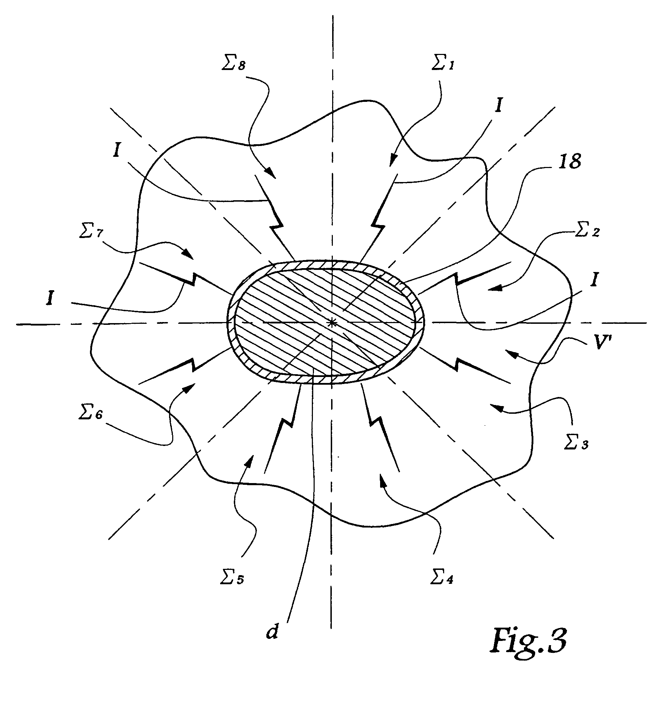

Comme il ressort plus particulièrement de la figure 3, la partie du volume V' entourant l'extrémité du doigt d est divisée en secteurs angulaires Σ₁ à Σ₈ dans chacun desquels se produisent des phénomènes d'ionisation I et dans chacun desquels on peut déterminer des valeurs caractéristiques U₂, U₃, θ et f₄ comme indiqué ci-dessus. En effet, en fonction de l'orientation du doigt d et du positionnement des terminaisons nerveuses, il est possible que les phénomènes d'ionisation se produisent dans ces secteurs à des instants distincts et pour des tensions différentes. Bien entendu, le nombre et la définition des secteurs Σ₁ à Σ₈ dépendent des choix effectués lors de la modélisation.

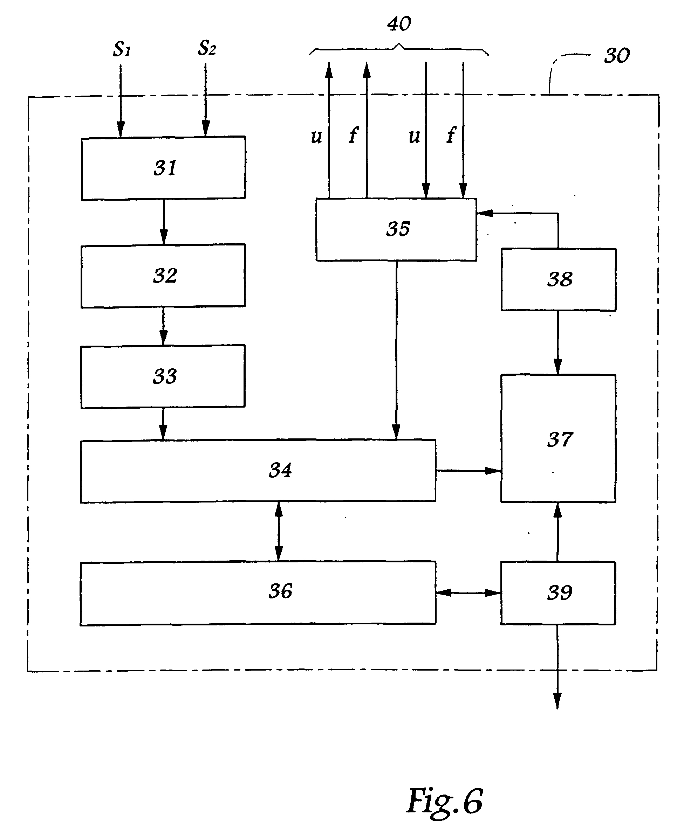

L'unité 30 est représentée schématiquement à la figure 6 et comprend un premier module 31 d'acquisition des signaux vidéo S₁ et S₂, un second module 32 de reconnaissance de formes permettant le localiser la zone d'appui de chaque doigt d sur le capteur 11 ou de chaque orteil o sur le capteur 12 et de définir les secteurs Σ₁ à Σ₈. Un module 33 de quantification de la luminance par zone permet d'analyser les signaux S₁ et S₂ en provenance du module 32, les signaux correspondant étant alors transmis à un calculateur 34 apte à effectuer les opérations logiques précédemment envisagées pour la détermination des valeurs caractéristiques U₂, U₃, Φ et f₄. Le calculateur 34 est relié à une interface 35 qui est raccordée au générateur 40 et permet de le commander et de connaître en permanence les valeurs de la tension et de la fréquence générées.

Le calculateur 34 est également relié à une base de données 36 dans laquelle sont stockées des valeurs de références des grandeurs caractéristiques pour des objets connus, ce qui permet une comparaison entre les grandeurs déterminées lors de chaque expérience et ces objets connus.

Un moniteur 37 est également prévu pour faire connaître à l'utilisateur le résultat des opérations effectuées par le calculateur 34, ce moniteur permettant de contrôler un module 38 de commande de l'interface 35.

Enfin, un dispositif 39 permet d'accéder à un réseau informatique interne ou externe pour la confrontation de données et/ou l'accès à des données de références supplémentaires.

L'ensemble de l'unité 30 peut être intégré dans un ordinateur incorporé dans le boîtier 1 ou relié à celui-ci par une liaison 44 adaptée.

Lorsque les signaux S₁ et S₂ correspondant aux différents secteurs Σ₁ à Σ₈ ont été traités, de préférence simultanément, pour chaque doigt d ou orteils de l'utilisateur, l'étape finale du procédé consiste à rattacher les valeurs caractéristiques détectées U₂, U₃, Φ et f₄ à chaque secteur Σ₁ à Σ₈, pour permettre une analyse quantitative et qualitative.

Dans le second mode de réalisation de l'invention représenté à la figure 7, les éléments analogues à ceux du premier mode de réalisation portent des références identiques augmentées de 100. Le capteur 111 de ce mode de réalisation comprend deux plaques isolantes 113 et 115 séparées par une entretoise 114 et définissant entre elles un volume V permettant de recevoir un électrolyte liquide pouvant être relié par un conducteur 141a d'une liaison 141 à un générateur non représenté du type du générateur 40. Un second conducteur 141c relie la borne opposée du générateur et le doigt d de l'utilisateur, ce qui permet d'appliquer directement une tension prédéterminée entre ce doigt et l'électrolyte 116. Le générateur fonctionne alors en mode commun et la mesure peut être limitée au seul doigt d, sans nécessité pour l'utilisateur de poser les pieds sur le capteur 12.

Le conducteur 141c peut être mis en contact avec le doigt d grâce à une pastille conductrice 141d du type utilisé en électro-cardiographie.

Selon une variante, le potentiel issu du générateur peut être appliqué par le conducteur 141c au poignet de l'utilisateur, celui-ci pouvant appliquer les cinq doigts de la main sur le capteur 111.

Du côté de la plaque 113 opposée au volume V est disposée une couche 117 de mousse de matière plastique transparente, par exemple à base de polymère, comprenant des micro-alvéoles dans lesquelles est emprisonné un gaz ou un mélange gazeux 119 du type du mélange 19. Un revêtement opaque 118 est disposé sur la couche 117 et isole cette couche de l'atmosphère ambiante en emprisonnant le gaz ou mélange 119. Le revêtement 118 est souple et constitue donc une membrane délimitant le volume V' de la couche 117 dans lequel est confiné le mélange 119.

Comme précédemment, un utilisateur peut déformer le revêtement 118 et la couche 117 lorsqu'il appuie l'extrémité d'un doigt d, ce qui a pour effet de châsser le mélange gazeux autour de ce doigt.

Comme précédemment, les phénomènes d'ionisation I peuvent être visualisées par transparence à travers les plaques 113 et 115 et l'électrolyte 116, qui sont transparents comme les plaques 13 et 15 et l'électrolyte 16.

Le capteur 11 du premier mode de réalisation peut être utilisé avec une alimentation en tension en mode commun, alors que le capteur 111 peut être utilisé avec un dispositif à alimentation en tension en mode différentiel, ceci résultant d'un choix de l'opérateur.

Quel que soit le mode de réalisation considéré, l'électrolyte 16 ou 116 constitue une première armature d'un condensateur dont la seconde armature est constituée par l'objet à étudier, par exemple le doigt d ou l'orteil d'un utilisateur.

La présente invention peut être utilisée pour la caractérisation de corps biologiques et, notamment, la détermination des propriétés biologiques d'un corps humain, animal ou végétal. Elle peut être utilisée par un praticien pour la détermination de méridiens au sens de l'acupuncture, le trajet d'écoulement d'un fluide énergétique, comme le sang d'un mammifère ou la sève d'un végétal, cette dernière application de l'invention permettant de contrôler les caractéristiques biologiques des plantes en vue de leur sélection.

L'invention permet en particulier de comparer les caractéristiques de plantes entre elles, par exemple les caractéristiques biologiques de plantes obtenues par des cultures de types différents, biologiques, intensifs ou transgéniques.

Une autre application particulièrement importante de l'invention concerne le contrôle dimensionnel, structurel ou de l'état de pièces mécaniques ou électriques, dans la mesure où des zones de décharges électriques plus concentrées peuvent être observées sur une pièce présentant des micro-fissures, des lignes de faiblesses ou des défauts de surface.

L'invention peut également être utilisée dans le domaine de l'hydrologie, par exemple pour une approche analytique de la composition de l'eau, et dans le domaine de la recherche fondamentale, notamment pour la différenciation de cellules de structure voisine.

## Revendications

1. Procédé de détermination des propriétés physiques d'un objet (m, p, d) au cours duquel :
- on dispose ledit objet au contact d'un capteur (11, 12 ; 111) dont un élément conducteur (16, 116) est électriquement connecté à un générateur de tension (40) ;
- on applique une tension alternative (U) entre ledit objet et ledit élément ;
- on visualise et/ou on enregistre un signal optique (S'₁, S'₂) représentatif de la quantité de lumière (L) d'ionisation d'au moins un gaz (19, 119) au voisinage dudit objet,
**caractérisé en ce qu'**il consiste à :
- augmenter progressivement ladite tension (U) jusqu'à une valeur finale (U₁) où une luminance maximum (L₁) est observée ;
- déterminer comme première valeur caractéristique dudit objet la valeur de ladite tension (U₂) à partir de laquelle la luminance (L₂) du signal est supérieure ou égale à environ 10% de la luminance maximum et
- déterminer comme seconde valeur caractéristique dudit objet la valeur de ladite tension (U₃) à partir de laquelle la luminance (L₃) du signal est supérieure ou égale à environ 90% de la luminance maximum.

2. Procédé selon la revendication 1, **caractérisé en ce qu'**il consiste à augmenter ladite tension par paliers (Pᵢ) , chaque palier correspondant à l'acquisition d'une image dudit signal (S'₁, S'₂).

3. Procédé selon l'une des revendications précédentes, **caractérisé en ce qu'**il consiste à déterminer, comme autre valeur caractéristique dudit objet (m, p, d) un angle de déphasage (Φ) entre la variation de luminance (L₁-L₃) et la variation de tension (U₀-U₃) dans le temps, ledit angle étant défini comme la différence de pente entre une première droite (D₁) représentative de l'augmentation de tension (U) dans le temps et une seconde droite représentative d'une évolution linéaire de la luminance dans le temps, entre des instants (A₂, A₃) où la luminance a des valeurs (L₂, L₃) respectivement égales à environ 10% et environ 90% de la luminance maximum (L₁).

4. Procédé selon l'une des revendications précédentes, **caractérisé en ce qu'**il consiste à augmenter ladite tension (U) par paliers (Pᵢ) et à faire varier la fréquence (f) de ladite tension sur chaque palier.

5. Procédé selon la revendication 4, **caractérisé en ce qu'**il consiste à déterminer, comme autre valeur caractéristique dudit objet, la valeur de la fréquence (f₄) de ladite tension (U) correspondant à une luminance relative maximum (L'₄) lorsque ladite fréquence (f) varie, alors que ladite tension est maintenue à une valeur de palier (U₄) correspondant à une luminance intermédiaire (L₄), notamment égale à environ 50% de la luminance maximum (L₁).

6. Procédé selon l'une des revendications précédentes, **caractérisé en ce qu'**il consiste à diviser une surface de mesure définie autour dudit objet (d) sur ledit capteur (11) en zones individuelles de mesure (Σ₁-Σ₈) et à déterminer lesdites valeurs caractéristiques (U₂, U₃, Φ, f₄) pour chaque zone individuelle de mesure.

7. Procédé selon l'une des revendications précédentes, **caractérisé en ce qu'**il consiste à mesurer les propriétés physiques d'un corps humain ou animal en appliquant simultanément les dix doigts (d) d'un sujet sur au moins un capteur (11) et les dix orteils (0) dudit sujet sur au moins un autre capteur (12), à alimenter les éléments conducteurs (16) de ces capteurs en mode différentiel à partir d'un générateur de tension (40) et à déterminer lesdites valeurs caractéristiques en parallèle pour les dix doigts et les dix orteils.

8. Installation destinée à la mise en oeuvre d'un procédé conforme à l'une des revendications précédentes pour la détermination des propriétés physiques d'un objet (m, p, d), ladite installation comportant :
- un capteur (11, 12, 111) comprenant une plaque électriquement isolante (13, 113) et un élément électriquement conducteur (16, 116) ;
- des moyens (40) d'alimentation électrique dudit élément électriquement conducteur et dudit objet avec une tension alternative variable (U) et
- des moyens (21, 22, 30) de visualisation et/ou d'enregistrement à travers ladite plaque et ledit élément conducteur, d'un signal optique (S₁, S₂) représentatif de la quantité de lumière (L) d'ionisation dudit objet, du fait de ladite tension appliquée,
**caractérisée en ce que** ledit capteur comprend une membrane souple (18, 118) délimitant, avec ladite plaque (13, 113), un volume (V') de confinement d'un gaz ou d'un mélange gazeux (19, 119) apte à s'ioniser sous l'effet de ladite tension (U),
**en ce que** ledit capteur est relié à un générateur (40) de façon à créer une tension (V) entre ledit objet (m, p, d) et ledit élément conducteur (16, 116) et
**en ce qu'**il est prévu des moyens (30) de traitement dudit signal optique (S₁, S₂) et de commande dudit générateur arrangés pour la mise en oeuvre d'un procédé selon l'une des revendications précédentes.

9. Installation selon la revendication 8, **caractérisée en ce que** ladite membrane souple (18, 118) est opaque.

10. Installation selon l'une des revendications 8 ou 9, **caractérisée en ce que** ledit gaz ou mélange gazeux (119) est emprisonné dans une mousse de matière plastique déformable (117).

11. Installation selon l'une des revendications 8 à 10, **caractérisée en ce qu'**elle comprend des moyens (32) de reconnaissance de forme, des moyens (33) de quantification de la luminance du signal observé (S'₁, S'₂) , des moyens de commande (38) desdits moyens d'alimentation électrique (40), des moyens de détermination automatique (34) de valeurs caractéristiques (U₂, U₃, Φ, f₄) dudit objet (m, p, d), des moyens de comparaison (36) avec des valeurs de références et/ou des moyens de visualisation (37) des résultats de la détermination ou de la comparaison.

## Patentansprüche

1. Verfahren zur Bestimmung von physikalischen Eigenschaften eines Objektes (m, p, d), bei dem:
- man das Objekt mit einem Aufnehmer (11,12,111) in Kontakt bringt, dessen leitendes Element (16,116) elektrisch mit einem Spannungsgenerator (40) verbunden ist;
- man eine Wechselspannung (U) zwischen das Objekt und das Element anlegt;
- man ein optisches Signal (S₁', S₂'), das die Menge an Ionisationslicht mindestens eines Gases (19,119) in der Nähe des Objektes darstellt, sichtbar macht und/oder aufzeichnet,
**dadurch gekennzeichnet, dass** es darin besteht:
- die Spannung (U) bis zu einem Endwert (U₁), bei dem die maximale Leuchtdichte (L₁) beobachtet wird, fortschreitend zu erhöhen;
- als ersten Kennwert des Objektes den Wert der Spannung (U₂) zu bestimmen, ab dem die Leuchtdichte (L₂) des Signals größer oder gleich ungefähr 10% der maximalen Leuchtdichte ist und
- als zweiten Kennwert des Objektes den Wert der Spannung (U₃) zu bestimmen, ab dem die Leuchtdichte (L₃) des Signals größer oder gleich ungefähr 90% der maximalen Leuchtdichte ist.

2. Verfahren nach Anspruch 1, **dadurch gekennzeichnet, dass** es darin besteht, die Spannung in Stufen (Pᵢ) zu erhöhen, wobei jede Stufe der Erfassung eines Bildes des Signals (S₁', S₂') entspricht.

3. Verfahren nach einem der vorhergehenden Ansprüche, **dadurch gekennzeichnet, dass** es darin besteht als anderen Kennwert des Objektes (m, p, d) einen Phasenverschiebungswinkel (Φ) zwischen der Änderung der Leuchtdichte (L₁-L₃) und der Änderung der Spannung (U₀-U₃) über die Zeit zu bestimmen, wobei der Winkel definiert ist als die Differenz der Steigung zwischen einer ersten Geraden (D₁), die repräsentativ für die Steigerung der Spannung (U) über die Zeit ist, und einer zweiten Geraden, die repräsentativ für eine lineare Entwicklung der Leuchtdichte über die Zeit ist, zwischen den Momenten (A₂, A₃), bei denen die Leuchtdichte Werte (L₂, L₃) aufweist, die jeweils ungefähr gleich 10% und ungefähr gleich 90% der maximalen Leuchtdichte (L₁) sind.

4. Verfahren nach einem der vorhergehenden Ansprüche, **dadurch gekennzeichnet, dass** es darin besteht, die Spannung (U) in Stufen (Pᵢ) zu erhöhen und die Frequenz (f) der Spannung auf jeder Stufe zu ändern.

5. Verfahren nach einem der vorhergehenden Ansprüche, **dadurch gekennzeichnet, dass** es darin besteht als anderen Kennwert des Objektes den Wert der Frequenz (f₄) der Spannung (U) entsprechend einer relativen maximalen Leuchtdichte (L'₄) zu bestimmen, wenn die Frequenz (f) variiert, wobei die Spannung auf einem Stufenwert (U₄) entsprechend
einer Zwischenleuchtdichte (L₄), nämlich gleich ungefähr 50% der maximalen Leuchtdichte (L₁) gehalten wird.

6. Verfahren nach einem der vorhergehenden Ansprüche, **dadurch gekennzeichnet, dass** es darin besteht, eine definierte Messfläche um das Objekt (d) herum auf den Aufnehmer (11) in individuelle Messzonen (Σ₁₋ Σ₈) aufzuteilen und die Kennwerte (U₂, U₃, δ, f₄) für jede individuelle Messzone zu bestimmen.

7. Verfahren nach einem der vorhergehenden Ansprüche, **dadurch gekennzeichnet, dass** es darin besteht, die physikalischen Eigenschaften eines menschlichen
oder tierischen Körpers zu messen, indem gleichzeitig die zehn Finger (d) eines Subjektes auf mindestens einen Aufnehmer (11) und die zehn Zehen (0) des Subjektes auf mindestens einen anderen Aufnehmer (12) aufgebracht werden, die leitenden Elemente (16) dieser Aufnehmer in differentieller Weise von einem Spannungsgenerator (40) zu versorgen und die Kennwerte für die zehn Finger und die zehn Zehen in paralleler Weise zu bestimmen.

8. Einrichtung zur Durchführung eines Verfahrens nach einem der vorhergehenden Ansprüche für die Bestimmung der physikalischen Eigenschaften eines Objektes (m, p, d), wobei die Einrichtung umfasst:
- einen Aufnehmer (11,12,111), der eine elektrisch isolierende Platte (13,113) und ein elektrisch leitendes Element (16,116) umfasst;
- Mittel (40) zur elektrischen Versorgung des elektrisch leitenden Elementes und des Objektes mit einer veränderlichen Wechselspannung (U) und
- Mittel (21,22,30) zur Sichtbarmachung und/oder Aufzeichnung über die Platte und das leitende Element eines optischen Signals (S₁, S₂), das für die Menge des Ionisationslichtes (L) des Objektes aufgrund der ausgelegten Spannung repräsentativ ist, **dadurch gekennzeichnet, dass** der Aufnehmer eine biegsame Membran (18,118) umfasst, die mit der Platte (13,113) ein Volumen (V')zum Einschließen eines Gases oder einer Gasmischung begrenzt, das oder die unter der Wirkung der Spannung (U) sich ionisieren kann,
dass der Aufnehmer mit einem Generator (40) verbunden ist, um eine Spannung (V) zwischen dem Objekt (m, p, d) und dem leitenden Element zu erzeugen und dass Mittel (30) zur Behandlung des optischen Signals (S₁, S₂) und zur Steuerung des Generators vorgesehen sind, die für die Durchführung eines Verfahrens nach einem der vorhergehenden Ansprüche angeordnet sind.

9. Einrichtung nach Anspruch 8, **dadurch gekennzeichnet, dass** die biegsame Membran opak ist.

10. Einrichtung nach einem der Ansprüche 8 oder 9, **dadurch gekennzeichnet, dass** das Gas oder die Gasmischung (119) in einem Schaum aus verformbarem Kunststoffmaterial (117) eingeschlossen ist.

11. Einrichtung nach einem der Ansprüche 8 bis 10, **dadurch gekennzeichnet, dass** sie Mittel (32) zur Formerkennung, Mittel (33) zur Quantifizierung der Leuchtdichte des beobachten Signals (S₁', S₂'), Mittel (38) zur Steuerung der Mittel (40) zur elektrischen Versorgung, Mittel (34) zur automatischen Bestimmung von Kennwerten (U₂, U3, Φ, f₄) des Objektes (m, p, d), Mittel (36) zum Vergleich mit Referenzwerten und/oder Mittel (37) zur Sichtbarmachung von Ergebnissen der Bestimmung oder des Vergleichs umfasst.

## Claims

1. Method for determining the physical properties of an object (m, p, d) during the course of which:
- the said object is placed in contact with a sensor (11, 12; 111) in which one conductive element (16, 116) is electrically connected to a voltage generator (40);
- an alternating voltage (U) is applied between the said object and the said element;
- an optical signal (S'₁, S'₂), representing the quantity of ionisation light (L) of at least one gas (19, 119) around the said object, is visualised and/or recorded,
**characterised in that** it consists in:
- progressively increasing the said voltage (U) until a final value (U₁) or a maximum luminance (L₁) is observed;
- determining, as the first characteristic value of the said object, the value of the said voltage (U₂) at which the luminance (L₂) of the signal is greater than or equal to about 10% of the maximum luminance and
- determining, as the second characteristic value of the said object, the value of the said voltage (U₃) at which the luminance (L₃) of the signal is greater than or equal to about 90% of the maximum luminance.

2. Method according to claim 1, **characterised in that** it consists in increasing the said voltage in steps (Pᵢ), each step corresponding to the acquisition of one image of the said signal (S'₁, S'₂).

3. Method according to one of the preceding claims, **characterised in that** it consists in determining, as another characteristic value of the said object (m, p, d), an angle of phase difference (Φ) between the temporal fluctuation in luminance (L₁-L₃) and the temporal fluctuation in voltage (U₀ - U₃), the said angle being defined as the difference in slope between a first line (D₁) representing the temporal increase in voltage (U) and a second line representing a temporal linear evolution of the light between the moments (A₂, A₃) when the luminance has the values (L₂, L₃) respectively equal to about 10% and about 90% of the maximum luminance (L₁).

4. Method according to one of the preceding claims, **characterised in that** it consists in increasing the said voltage (U) in steps (Pᵢ) and in varying the frequency (f) of the said voltage at each step.

5. Method according to claim 4, **characterised in that** it consists in determining, as another characteristic value of the said object, the value of the frequency (f₄) of the said voltage (U) corresponding to a relative maximum luminance (L'₄) when the said frequency (f) varies, whilst the said voltage is maintained at a step value (U₄) corresponding to an intermediate luminance (L₄), in particular equal to about 50% of the maximum luminance (L₁).

6. Method according to one of the preceding claims, **characterised in that** it consists in dividing a measuring surface defined around the said object (d) on the said sensor (11) into individual measuring zones (Σ₁- Σ₈) and in determining the said characteristic values (U₂, U₃, Φ, f₄), for each individual measuring zone.

7. Method according to one of the preceding claims, **characterised in that** it consists in measuring the physical properties of a human or animal body by applying the ten fingers (d) of a subject simultaneously on to at least one sensor (11) and the ten toes (0) of the said subject on to at least one other sensor (12), in supplying the conductive elements (16) of these sensors in differential mode from a voltage generator (40) and in determining the said characteristic values in parallel for the ten fingers and the ten toes.

8. Apparatus designed for the implementation of a method in accordance with one of the preceding claims for determining the physical properties of an object (m, p, d), the said apparatus comprising:
- a sensor (11, 12, 111) comprising an electrically insulating plate (13, 113) and an electrically conductive element (16, 116);
- electric supply means (40) for the said electrically conductive element and the said object with an alternative variable voltage (U) and
- visualisation and/or registration means (21, 22, 30) across the said plate and the said conductive element, for an optical signal (S₁, S₂) representing the quantity of ionisation light (L) of the said object, resulting from the said applied voltage,
**characterised in that** the said sensor includes a pliable membrane (18, 118) delimiting, with the said plate (13, 113), a confining volume (V') of a gas or of a gaseous mixture (19, 119) capable of ionising under the effect of the said voltage (U),
**in that** the said sensor is linked to a generator (40) so as to create a voltage (V) between the said object (m, p, d) and the said conductive element (16, 116) and
**in that** it is provided with suitable means (30) for processing the said optical signal (S₁, S₂) and controlling the said generator for the implementation of a method according to one of the preceding claims.

9. Apparatus according to claim 8, **characterised in that** the said pliable membrane (18, 118) is opaque.

10. Apparatus according to one of claims 8 or 9, **characterised in that** the said gas or gaseous mixture (119) is confined in a deformable cellular plastic material (117).

11. Apparatus according to one of claims 8 to 10, **characterised in that** it comprises form recognition means (32), means (33) for quantifying the luminance of the signal observed (S'₁, S'₂), control means (38) for the said electric supply means (40), means for the automatic determination (34) of the characteristic values (U₂, U₃, Φ, f₄) of the said object (m, p, d), means for making comparisons (36) with base values and/or means for visualising (37) the results of the determination or of the comparison.
